# EUROPÄISCHE PATENTSCHRIFT

(11) **EP 2 654 478 B1**
(45) Veröffentlichungstag und Bekanntmachung des Hinweises auf die Patenterteilung: **15.08.2018**
(21) Anmeldenummer: 11799689.2
(22) Anmeldetag: 19.12.2011
(51) Int. Cl.: A43B 7/14, A43B 3/00

(54) **SCHUTZEINRICHTUNG FÜR DEN DIABETISCHEN FUß UND VERFAHREN ZUM SCHUTZ DES DIABETISCHEN FUßES VOR FEHLENDER DURCHBLUTUNG**
PROTECTIVE DEVICE FOR A DIABETIC FOOT AND METHOD FOR PROTECTING THE DIABETIC FOOT FROM LACK OF CIRCULATION
DISPOSITIF DE PROTECTION POUR LE PIED DIABÉTIQUE ET PROCÉDÉ DE PROTECTION DU PIED DIABÉTIQUE CONTRE UNE INSUFFISANCE DE LA CIRCULATION SANGUINE

(30) Priorität: 21.12.2010 DE 102010063740; 21.12.2010 DE 202010013176 U
(43) Veröffentlichungstag der Anmeldung: 30.10.2013
(73) Patentinhaber: mediXmind GmbH, 39114 Magdeburg (DE)
(72) Erfinder: MERTENS, Peter R., 39122 Magdeburg (DE); KLOSE, Silke, 39112 Magdeburg (DE); MOTZKAU, Markus, 39116 Magdeburg (DE)
(74) Vertreter: Kailuweit & Uhlemann Patentanwälte Partnerschaft mbB
(86) Internationale Anmeldenummer: PCT/EP2011/073227
(87) Internationale Veröffentlichungsnummer: WO 2012/084814

(56) Entgegenhaltungen:
- US-A- 5 566 479
- US-A- 5 642 096
- US-A- 5 929 332
- US-A1- 2008 109 183

## Beschreibung

Die Erfindung betrifft Schutzeinrichtungen für den diabetischen Fuß und Verfahren zum Schutz des diabetischen Fußes vor fehlender Durchblutung.

Eine Folgewirkung von Diabetes ist ein Gefühlsverlust insbesondere im Fuß. Dadurch können Druckstellen entstehen, die zu Wunden führen, ohne dass dies der Patient merkt. Zur Vorbeugung und Kontrolle sind verschiedene Einrichtungen bekannt, um einer schädigenden Druckbelastung vorzubeugen.

Durch die Druckschrift DE 92 03 788 U1 ist eine Schuheinlage zur Ulcusprohylaxe am diabetischen Fuß bekannt. Dazu ist in einer Einlegesohle, welche sich ganz oder größtenteils entlang der Fußsohle erstreckt, im Bereich des Vorfußes eine kompressible Hydrozelle aus flexiblem Kunststoffmaterial angeordnet. Weiterhin steht ein Sensor zur Druckerfassung mit der Flüssigkeit in der Hydrozelle in Verbindung. Darüber hinaus ist der Sensor mit einem Zeitschalter verbunden, wobei der Sensor sowohl beim Überschreiten eines einstellbaren Maximaldruck-Schwellwerts als auch bei Erreichen eines unter diesem Maximalwert liegenden Druckschwellenwerts, sofern dieser über eine bestimmte Zeitdauer erreicht wird, ein Signal an einen Warnsignalgeber gibt. Die Druckmessung ist dabei auf einen engen Bereich der Fußsohle begrenzt. Dabei ist es schwierig die Maximaldruckschwellenwert und Zeitdauern festzulegen, weil sie nicht nur patientenabhängig sind, sondern auch für verschiedene Bereiche eines Fußes unterschiedlich sein können, zum Beispiel infolge von Läsionen.

Die Druckschrift US 4,647,918 A beschreibt eine Einrichtung zur Erfassung der Druckbelastung an mehreren Stellen des Fußes. Dazu wird eine Schuheinlage mit mehreren Drucksensoren verwendet. Die Drucksensoren sind an verschiedenen Stellen des Fußes platziert. Eine Signalisierung erfolgt mittels eines Geräts, welches zum einen mit den Sensoren verbunden und zum anderen durch den Patienten tragbar ist. Die Auswertung erfolgt zeitbezogen über einen Mikroprozessor, der über einen Kabelbaum elektrisch leitend mit den Messwertgebern verbunden ist, entsprechend vorgegebener und gespeicherter Grenzwerte. Eine Lichtquelle dient beispielsweise der Signalisierung. Der Kabelbaum muss dabei von der Schuheinlage über den Schuh und das Bein zum Gerät geführt werden. Dabei sind durch die Kabel hervorgerufene und nicht bestimmte Druckbelastungen am Fuß nicht auszuschließen. Insbesondere bei der Bewegung kann dieser im Schuh am Fuß reiben und zu Verletzungen führen. Das ist nur durch besondere Maßnahmen in der Fußbekleidung vermeidbar.

Durch die Druckschrift US 5 642 096 A ist eine Vorrichtung zur Vorbeugung von Geschwüren an den Füßen von Diabetes-Patienten bekannt. Dazu ist ein Sensor in einer diesen umhüllenden Flüssigkeit in einer Schuhinnensohle angeordnet. Mittels des Sensors werden der Druck und die Temperatur eines Bereichs der Fußsohle erfasst. Der Sensor ist mit einem Signalgenerator verbunden, so dass das Überschreiten von vorgegebenen Grenzwerten den Patienten signalisiert wird.

Die Druckschrift US 2008/109183 A1 beinhaltet ein Gerät zur Messung der Temperatur, des Druckes und/oder der Feuchtigkeit des Fußes eines Patienten an einer Stelle oder mehreren Stellen des Fußes. Der jeweilige Sensor ist dazu ein Bestandteil der Innensohle eines Schuhs oder einer Socke. Der Sensor oder die Sensoren sind mit einem Datenverarbeitungssystem zur Warnung des Patienten verbunden.

Die Druckschrift US 5 566 479 A bezieht sich auf einen Schuh für Patienten, wobei ein übermäßiger Druck detektiert wird. Dazu wird der Druck an verschiedenen Stellen überwacht. Bei Erreichen eines Schwellwerts des Drucks wird der Träger des Schuhs über ein Datenverarbeitungssystem gewarnt. Die Temperatur wird nicht erfasst, aus dem Druck wird auf die Temperatur geschlossen.

Die Druckschrift US 5 929 332 A zeigt einen Schuh mit Sensoren an den Außenbereichen der Schuhsohle für die Fußsohle des Trägers. Die gezeigten Sensoren sind Bestandteile einer Innensohle des Schuhs, die die Innensohle aber nicht überragen. Die Sensoren sind mit einem Computer verbunden, so dass ein Überschreiten von Grenzwerten signalisiert wird.

Mittels dieser Einrichtungen werden Drücke und/oder Temperaturen der Fußsohle erfasst.

Der in den Patentansprüchen 1 und 12 angegebenen Erfindung liegt die Aufgabe zugrunde, einen den diabetischen Fuß schädigenden Druck einfach und ökonomisch günstig zu vermeiden.

Diese Aufgabe wird mit den in den Patentansprüchen 1 und 12 aufgeführten Merkmalen gelöst.

Die Schutzeinrichtungen für den diabetischen Fuß und die Verfahren zum Schutz des diabetischen Fußes vor fehlender Durchblutung zeichnen sich insbesondere dadurch aus, dass ein den diabetischen Fuß schädigender Druck einfach und ökonomisch günstig zu vermeiden ist.

Dazu besteht die Einrichtung aus einer Sohle mit Seitenteilen jeweils mit Druck- und Temperatursensoren für Bereiche der Fußsohle zur Messung vertikaler Belastungen und für seitliche Bereiche des Fußes zur Messung horizontaler Belastungen einschließlich der der Durchblutung zuordenbaren Temperaturen der jeweiligen Bereiche des Fußes. Weiterhin sind die Druck- und Temperatursensoren mit einem Datenverarbeitungssystem zum Vergleich der Messwerte mit gespeicherten Grenzwerten zusammengeschaltet. Darüber hinaus ist das Datenverarbeitungssystem mit einem Sender zusammengeschaltet. Die Druck- und Temperatursensoren, das Datenverarbeitungssystem und der Sender sind Bestandteile der Sohle. Das Datenverarbeitungssystem und der Sender sind mit einer Energiequelle entweder als Bestandteil der Sohle oder mit einer Befestigungseinrichtung für ein Körperteil verbunden.

Zum Schutz des diabetischen Fußes vor fehlender Durchblutung erfolgt damit
- eine Messung des auf die Fußsohle und auf die seitlichen Bereiche des Fußes wirkenden Druckes mit Drucksensoren in einer Sohle und daran befestigten Seitenteilen und
- eine gleichzeitige Erfassung der jeweiligen Durchblutung zuordenbaren Temperaturen mittels Temperatursensoren.

Das Datenverarbeitungssystem und der Sender sind so miteinander verbunden, dass
- eine Signalisierung erfolgt, wenn wenigstens an einem Drucksensor ein Druckwert als ein Grenzdruckwert über einen Zeitraum überschritten und/oder wenigstens an einem Temperatursensor die Temperatur sinkt und
- die Signalisierung nicht erfolgt, wenn an diesem Drucksensor der Druck unter den Druckwert sinkt und/oder an diesem Temperatursensor die Temperatur mit der Durchblutung steigt.

Eine Person mit Diabetes in ihrer schweren Ausbildung hat im Fuß kein Gefühl. Deshalb besteht die Gefahr, dass er mit seinem Fuß eine Position einnimmt, die an einem oberflächlichen Ort des Fußes permanent einen Druck hervorruft. Das passiert in der Regel in Verbindung mit einer Fußbekleidung. Diesbezüglich sind vor allem die Fußsohle und die seitlichen Bereiche des Fußes gefährdet.

Der permanent auf die Fußoberfläche ausgeübte Druck bewirkt eine Unterbrechung des Blutflusses in den Gefäßkapillaren, wodurch die betroffenen Gewebebereiche abkühlen. Die fehlende Durchblutung führt zu Verletzungen der betroffenen Bereiche und kann das Absterben von Gewebebereichen bewirken. Häufig wird in der Folge eine Entfernung von Zehen, zum Teil des gesamten Fußes unumgänglich. Bei fortbestehender Druckbelastung verheilen Wunden zudem sehr langsam.

Die Schutzeinrichtung zeichnet sich insbesondere dadurch aus, dass sie auch in normalen Schuhen als Fußbekleidung tragbar ist. Dabei kann die die Schutzeinrichtung tragende Person auf einen zu lange anhaltenden Druck über den Sender aufmerksam gemacht werden. Die Person ist damit in der Lage, die Haltung oder die Stellung des Fußes zu verändern, um übermäßige Druckbelastungen bestimmter Fußbereiche zu vermeiden.

Dazu besitzt die Schutzeinrichtung die Druck- und Temperatursensoren, die mit dem Datenverarbeitungssystem zur Auswertung zusammengeschaltet sind. Die Druck- und Temperatursensoren sind entweder jeweils separate Sensoren oder befinden sich zusammen auf einem gemeinsamen Substrat als kompakter Sensor. Vorteilhafterweise ist eine Energiequelle mit diesen verbunden, die gleichzeitig ein Bestandteil der Sohle ist oder extern mittels der Befestigungseinrichtung an einem Körperteil oder einem Kleidungsstück fixierbar ist. Damit ist eine autarke und kompakte Messwerterfassung und Auswertung in Form der Schutzeinrichtung gegeben. Als Drucksensoren können beispielsweise piezoresistive oder kapazitive Drucksensoren eingesetzt werden. Temperatursensoren sind beispielsweise bekannte Widerstandsthermometer.

Vorteilhafte Ausgestaltungen der Erfindung sind in den Patentansprüchen 2 bis 11 und 13 angegeben.

Nach der Weiterbildung des Patentanspruchs 2 besteht die Sohle vorteilhafterweise
- aus einem Träger für wenigstens Druck- und Temperatursensoren, das Datenverarbeitungssystem, den Sender und elektrischen Leitern und
- aus einem Deckkörper auf dem Träger, Druck- und Temperatursensoren, dem Datenverarbeitungssystem, dem Sender und elektrischen Leitern.

Weiterhin bestehen die Seitenteile aus dem Träger für wenigstens Druck- und Temperatursensoren und elektrischen Leitern und aus dem Deckkörper auf dem Träger, Druck- und Temperatursensoren und elektrischen Leitern.

Insbesondere ist der Deckkörper günstigerweise ein elastischer Körper, in dem die Druck- und Temperatursensoren, das Datenverarbeitungssystem, der Sender und die elektrischen Leiter eingebettet sind.

Der Träger und der Deckkörper bestehen nach der Weiterbildung des Patentanspruchs 3 aus einem Kunststoff und/oder sind Textilien und/oder Leder.

Der Träger und der Deckkörper sind nach der Weiterbildung des Patentanspruchs 4 miteinander vernäht oder verklebt. Das sind einfache und bekannte Verbindungstechnologien.

In Fortführung sind nach der Weiterbildung des Patentanspruchs 5 die den Träger und die den Deckkörper miteinander verbindende Nähte oder Klebestellen so ausgebildet und angeordnet, dass eine Naht oder eine Klebung einen Sensor als Druck- und/oder Temperatursensor positioniert. Vorteilhafterweise umschließt eine Naht und/oder Klebung den jeweiligen Sensor. Damit sind diese Bestandteile der Schutzeinrichtung einfach fixiert.

Die Energiequelle ist nach der Weiterbildung des Patentanspruchs 6 ein Akkumulator. Dieser ist weiterhin mit einem Steckverbinder zur lösbaren Verbindung mit einem Ladegerät verbunden. Damit ist der Akkumulator bei Nichtbenutzung der Schutzeinrichtung für eine weitere Benutzung aufladbar.

Der Akkumulator ist nach der Weiterbildung des Patentanspruchs 7 über ein Ladegerät mit einer mechanische Energie oder Bewegungsenergie in elektrische Energie wandelnden Einrichtung verbunden. Letztere ist günstigerweise in die Sohle integriert, so dass eine ständige Energieversorgung sichergestellt ist.

Das Datenverarbeitungssystem und der Sender sind nach der Weiterbildung des Patentanspruchs 8 so miteinander verbunden, dass bei Unter- und oder Überschreiten wenigstens eines Grenzwertes der Sender wenigstens ein Signal in Form elektromagnetischer Strahlung an einen Empfänger zur Signalisierung sendet. Durch die drahtlose Verbindung ist der Empfänger leicht an den verschiedensten Stellen des Körpers der Person platzier- und tragbar. Vorteilhafterweise erfolgt dabei die Signalisierung entsprechend des betroffenen Bereiches des Fußes, so dass die Person gezielt ihre Position verändern kann. Dazu werden verschiedene Signale genutzt.

Das Datenverarbeitungssystem und der Sender sind nach der Weiterbildung des Patentanspruchs 9 so miteinander verbunden, dass eine Signalisierung erfolgt, wenn wenigstens an einem Drucksensor ein oberer Grenzdruckwert über einen Zeitraum überschritten ist und nachfolgend an diesem Drucksensor keine Druckentlastung unter einen unteren Grenzwert über einen Zeitraum vorhanden ist.

Der Empfänger ist nach der Weiterbildung des Patentanspruchs 10 mit wenigstens einem Signalgeber für akustische Signale, optische Signale, mechanische Signale oder einer Kombination davon verbunden.

Nach der Weiterbildung des Patentanspruchs 11 ist ein Sensor zur Messung der Umgebungstemperatur mit dem Datenverarbeitungssystem verbunden, wobei sich dieser Sensor in einem Gehäuse befindet, das über ein elastisches Verbindungsteil mit der Sohle verbunden ist. Damit erfolgt eine Signalisierung in Abhängigkeit der Außentemperatur. Dieser Sensor ragt dazu einfach aus der Fußbekleidung.

Die Messwerte werden nach der Weiterbildung des Patentanspruchs 13 mit dem mit den Druck- und Temperatursensoren verbundenen Datenverarbeitungssystem mit gespeicherten Werten als Grenzdruckwerte im Datenverarbeitungssystem verglichen. Dabei erfolgt bei Überschreiten mindestens eines Grenzdruckwertes über einen Zeitraum verbunden mit sinkender Temperatur an wenigstens einem der Druck- und Temperatursensoren eine Signalisierung.

Ein Ausführungsbeispiel der Erfindung ist in den Zeichnungen jeweils prinzipiell dargestellt und wird im Folgenden näher beschrieben.
Es zeigen:
Fig. 1 eine Schutzeinrichtung für den diabetischen Fuß,
Fig. 2 eine Sohle mit Seitenteilen einer Schutzeinrichtung und
Fig. 3 eine Schnittdarstellung einer Sohle.

Im nachfolgenden Ausführungsbeispiel werden eine Schutzeinrichtung für den diabetischen Fuß und ein Verfahren zum Schutz des diabetischen Fußes vor fehlender Durchblutung zusammen näher erläutert.

Eine Schutzeinrichtung für den diabetischen Fuß besteht im Wesentlichen aus einer Sohle 1 mit Seitenteilen 2, Sensoren 3 als Druck- und Temperatursensoren 3, einem Datenverarbeitungssystem 4, einem Sender 5 und einer Energiequelle 6, einem Empfänger 7 und einem Signalgeber 8.

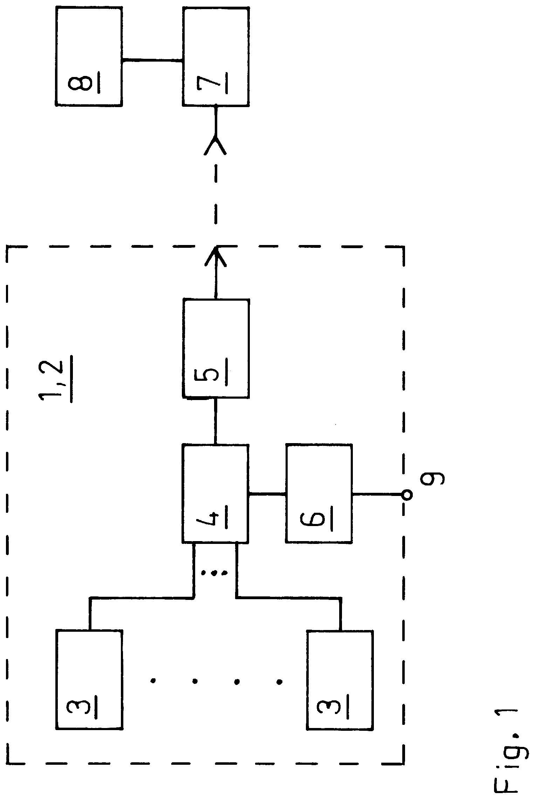

Die Fig. 1 zeigt eine Schutzeinrichtung für den diabetischen Fuß in einer prinzipiellen Darstellung.

Das Datenverarbeitungssystem 4 ist mit den Druck- und Temperatursensoren 3 für Bereiche der Fußsohle zur Messung vertikaler Belastungen und für seitliche Bereiche des Fußes zur Messung horizontaler Belastungen einschließlich der der Durchblutung zuordenbaren Temperaturen der jeweiligen Bereiche des Fußes zusammengeschaltet. Die Sensoren 3 sind dabei sowohl jeweils einzeln als auch auf einem Substrat zusammen einsetzbar. Das Datenverarbeitungssystem 4 ist weiterhin mit dem Sender 5 verbunden.

Im Datenverarbeitungssystem 4 erfolgt der Vergleich der Messwerte mit gespeicherten Grenzwerten. Eine Signalisierung erfolgt, wenn wenigstens an einem Drucksensor 3 ein oberer Grenzdruckwert über einen Zeitraum überschritten ist und nachfolgend an diesem Drucksensor 3 keine Druckentlastung unter einen unteren Grenzwert über einen weiteren Zeitraum vorhanden ist.

Der Empfänger 7 ist mit wenigstens einem Signalgeber 8 für akustische Signale, optische Signale, mechanische Signale oder einer Kombination davon verbunden. Die Verbindung basiert auf elektromagnetischen Strahlen. Besonders vorteilhaft ist ein akustischer Signalgeber, der beispielsweise im oder am Ohr tragbar ist. Aber auch ein am Körper getragener Vibrator als Signalgeber 8 für mechanische Signale ist vorteilhaft einsetzbar und gewährleistet eine sichere Signalisierung.

Das Datenverarbeitungssystem 4 und der Sender 5 sind mit einer Energiequelle 6 in Form eines Akkumulators 6 verbunden. Letzterer ist entweder ein Bestandteil der Sohle 1 oder mit einer Befestigungseinrichtung für ein Körperteil oder Kleidungsstück versehen und damit extern tragbar. Die Verbindung erfolgt über bekannte Kabel.

Zum Laden des Akkumulators 6 weist die Sohle 1 einen Steckverbinder 9 auf, so dass ein externes Ladegerät anschließbar ist. Damit ist der Akkumulator 6 bei Nichtbenutzung der Schutzeinrichtung aufladbar.

Weitere Bestandteile oder Bestandteile der Sohle 1 sind die Druck- und die Temperatursensoren 3, das Datenverarbeitungssystem 4 und der Sender 5.

Die Sohle 1 besteht aus einem Träger 10 für wenigstens die Druck- und Temperatursensoren 3, das Datenverarbeitungssystem 4, den Sender 5 und elektrischen Leitern und aus einem Deckkörper 11 auf dem Träger 10, den Druck- und den Temperatursensoren 3, dem Datenverarbeitungssystem 4, dem Sender 5 und den elektrischen Leitern.

Die Seitenteile 2 bestehen aus dem Träger 10 für die Druck- und die Temperatursensoren 3 und die elektrischen Leiter und aus dem Deckkörper 11 auf dem Träger 10, den Druck- und Temperatursensoren 3 und den elektrischen Leitern.

Als elektrische Leiter werden bekannte Kabel verwendet.

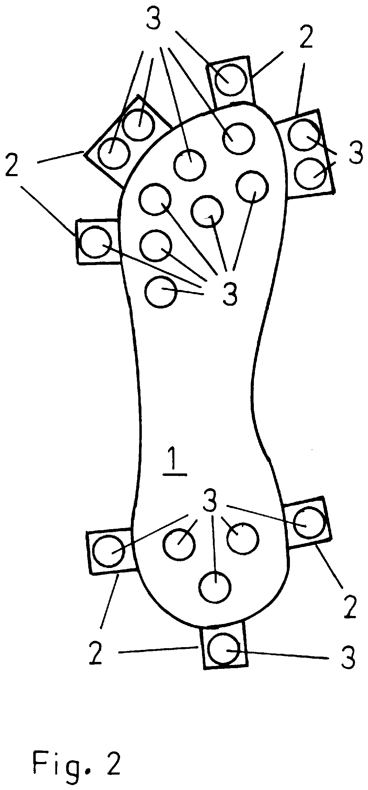

Die Fig. 2 zeigt eine Sohle mit Seitenteilen einer Schutzeinrichtung in einer prinzipiellen Darstellung.

Der Träger 10 und der Deckkörper 11 sind beispielsweise Textilien, die miteinander vernäht oder verklebt sind. Dabei sind die den textilen Träger 10 und die den textilen Deckkörper 11 miteinander verbindenden Nähte oder Klebungen so ausgebildet und angeordnet, dass eine Naht oder eine Klebung mindestens einen Sensor 3 positioniert.

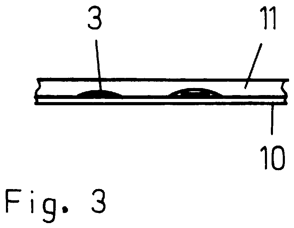

Die Fig. 3 zeigt eine prinzipielle Schnittdarstellung einer Sohle.

Zum Schutz des diabetischen Fußes vor fehlender Durchblutung wird mittels der Drucksensoren 3 in der Sohle 1 und daran befestigten Seitenteilen 2 der auf die Fußsohle und auf die seitlichen Bereiche des Fußes der Druck gemessen. Gleichzeitig werden mittels der Temperatursensoren 3 der jeweiligen Durchblutung zuordenbare Temperaturen erfasst. Die Messwerte werden mit dem mit den Druck- und Temperatursensoren 3 verbundenen Datenverarbeitungssystem 4 verglichen, wobei bei steigendem Druck verbunden mit sinkender Temperatur über einen Zeitraum an wenigstens einem der Druck- und Temperatursensoren 3 eine Signalisierung erfolgt.

Die Messwerte können mit dem mit den Druck- und Temperatursensoren 3 verbundenen Datenverarbeitungssystem 4 mit gespeicherten Werten als Grenzdruckwerte im Datenverarbeitungssystem 4 verglichen werden, wobei bei Überschreiten mindestens eines Grenzdruckwertes über einen Zeitraum verbunden mit sinkender Temperatur an wenigstens einem der Druck- und Temperatursensoren 3 eine Signalisierung erfolgt.

In einer Ausführungsform der Schutzeinrichtung ist der Akkumulator 6 über ein Ladegerät mit einem mechanische Energie oder Bewegungsenergie in elektrische Energie wandelnden Einrichtung verbunden.

In einer weiteren Ausführungsform der Schutzeinrichtung ist ein Sensor zur Messung der Umgebungstemperatur mit dem Datenverarbeitungssystem 4 verbunden, wobei sich dieser Sensor in einem Gehäuse befindet, das über ein elastisches Verbindungsteil mit der Sohle 1 verbunden ist.

## Patentansprüche

1. Schutzeinrichtung für den diabetischen Fuß mit den folgenden Merkmalen
- einer Sohle (1) mit daran befestigten Seitenteilen (2) jeweils mit Druck- und Temperatursensoren (3) für Bereiche der Fußsohle zur Messung vertikaler Belastungen und für seitliche Bereiche des Fußes zur Messung horizontaler Belastungen einschließlich der der Durchblutung zuordenbaren Temperaturen der jeweiligen Bereiche des Fußes,
- einem mit den Druck- und Temperatursensoren (3) zusammengeschaltetem Datenverarbeitungssystem (4) zum Vergleich der Messwerte mit gespeicherten Grenzwerten, wobei eine Signalisierung erfolgt, wenn wenigstens an einem Drucksensor (3) ein Druckwert als ein Grenzdruckwert über einen Zeitraum überschritten und/oder wenigstens an einem Temperatursensor (3) die Temperatur sinkt und dass die Signalisierung nicht erfolgt, wenn an diesem Drucksensor (3) der Druck unter den Druckwert sinkt und/oder an diesem Temperatursensor (3) die Temperatur mit der Durchblutung steigt,
- einem mit dem Datenverarbeitungssystem (4) zusammengeschaltetem Sender (5), wobei Druck- und Temperatursensoren (3), das Datenverarbeitungssystem (4) und der Sender (5) Bestandteile der Sohle (1) und Druck- und Temperatursensoren (3) Bestandteile der Seitenteile (2) sind, und
- einer mit dem Datenverarbeitungssystem (4) und dem Sender (5) verbundenen Energiequelle (6) entweder als Bestandteil der Sohle (1) oder mit einer Befestigungseinrichtung für ein Körperteil oder Kleidungsstück.

2. Schutzeinrichtung nach Patentanspruch 1, **dadurch gekennzeichnet, dass** die Sohle (1) aus einem Träger (10) für wenigstens Druck- und Temperatursensoren (3), das Datenverarbeitungssystem (4), den Sender (5) und elektrischen Leitern und aus einem Deckkörper (11) auf dem Träger (10), Druck- und Temperatursensoren (3), dem Datenverarbeitungssystem (4), dem Sender (5) und elektrischen Leitern besteht und dass die Seitenteile (2) aus dem Träger (10) für wenigstens Druck- und Temperatursensoren (3) und elektrischen Leitern und aus dem Deckkörper (11) auf dem Träger (10), Druck- und Temperatursensoren (3) und elektrischen Leitern bestehen.

3. Schutzeinrichtung nach Patentanspruch 2, **dadurch gekennzeichnet, dass** der Träger (10) und der Deckkörper (11) aus einem Kunststoff bestehen und/oder Textilien und/oder Leder sind.

4. Schutzeinrichtung nach Patentanspruch 2 und 3, **dadurch gekennzeichnet, dass** der Träger (10) und der Deckkörper (11) miteinander vernäht oder verklebt sind.

5. Schutzeinrichtung nach Patentanspruch 4, **dadurch gekennzeichnet, dass** die den Träger (10) und die den Deckkörper (11) miteinander verbindende Nähte oder Klebstellen so ausgebildet und angeordnet sind, dass eine Naht oder Klebung einen Sensor (3) als Druck- und/oder Temperatursensor (3) positioniert.

6. Schutzeinrichtung nach Patentanspruch 1, **dadurch gekennzeichnet, dass** die Energiequelle (6) ein Akkumulator (6) ist und dass der Akkumulator (6) mit einem Steckverbinder (9) zur lösbaren Verbindung mit einem Ladegerät verbunden ist.

7. Schutzeinrichtung nach Patentanspruch 6, **dadurch gekennzeichnet, dass** der Akkumulator (6) über ein Ladegerät mit einem mechanische Energie oder Bewegungsenergie in elektrische Energie wandelnden Einrichtung verbunden ist.

8. Schutzeinrichtung nach Patentanspruch 1, **dadurch gekennzeichnet, dass** das Datenverarbeitungssystem (4) und der Sender (5) so miteinander verbunden sind, dass bei Unter- und oder Überschreiten wenigstens eines Grenzwertes der Sender (5) wenigstens ein Signal in Form elektromagnetischer Strahlung an einen Empfänger (7) zur Signalisierung sendet.

9. Schutzeinrichtung nach Patentanspruch 8, **dadurch gekennzeichnet, dass** das Datenverarbeitungssystem (4) und der Sender (5) so miteinander verbunden sind, dass eine Signalisierung erfolgt, wenn wenigstens an einem Drucksensor (3) ein oberer Grenzdruckwert über einen Zeitraum überschritten ist und nachfolgend an diesem Drucksensor (3) keine Druckentlastung unter einen unteren Grenzwert über einen Zeitraum vorhanden ist.

10. Schutzeinrichtung nach Patentanspruch 8, **dadurch gekennzeichnet, dass** der Empfänger (7) mit wenigstens einem Signalgeber (8) für akustische Signale, optische Signale, mechanische Signale oder einer Kombination davon verbunden ist.

11. Schutzeinrichtung nach Patentanspruch 1, **dadurch gekennzeichnet, dass** ein Sensor zur Messung der Umgebungstemperatur mit dem Datenverarbeitungssystem (4) verbunden ist, wobei sich dieser Sensor in einem Gehäuse befindet, das über ein elastisches Verbindungsteil mit der Sohle mechanisch verbunden ist.

12. Verfahren zum Schutz des diabetischen Fußes vor fehlender Durchblutung, wobei mit Drucksensoren (3) in einer Sohle (1) und daran befestigten Seitenteilen (2) der auf die Fußsohle und auf die seitlichen Bereiche des Fußes der Druck gemessen wird, wobei gleichzeitig mittels von Temperatursensoren (3) der jeweiligen Durchblutung zuordenbare Temperaturen erfasst werden und wobei die Messwerte mit einem mit den Druck- und Temperatursensoren (3) verbundenen Datenverarbeitungssystem (4) verglichen werden, wobei bei steigendem Druck verbunden mit sinkender Temperatur über einen Zeitraum an wenigstens einem der Druck- und Temperatursensoren (3) eine Signalisierung erfolgt.

13. Verfahren nach Patentanspruch 12, wobei dass die Messwerte mit dem mit den Druck- und Temperatursensoren (3) verbundenen Datenverarbeitungssystem (4) mit gespeicherten Werten als Grenzdruckwerte im Datenverarbeitungssystem (4) verglichen werden, so dass bei Überschreiten mindestens eines Grenzdruckwertes über einen Zeitraum verbunden mit sinkender Temperatur an wenigstens einem der Druck- und Temperatursensoren (3) eine Signalisierung erfolgt.

## Claims

1. Protective device for a diabetic foot, comprising the following features:
- a sole (1) having lateral elements (2) secured thereto, which each have pressure and temperature sensors (3) for measuring vertical loads in the case of foot sole regions, and for measuring horizontal loads in the case of lateral regions of the foot, as well as for measuring the temperatures of the respective regions of the foot, which temperatures can be associated with blood circulation,
- a data-processing system (4) connected to the pressure and temperature sensors (3) in order to compare the measured values with stored limit values, wherein signalling takes place if a pressure value, as a limit pressure value, at least at one pressure sensor (3) is exceeded over a time period and/or the temperature falls at least at one temperature sensor (3), and signalling does not take place if the pressure falls below the pressure value at said pressure sensor (3) and/or if the temperature increases at said temperature sensor (3) as the blood circulation increases,
- a transmitter (5) connected to the data-processing system (4), wherein pressure and temperature sensors (3), the data-processing system (4) and the transmitter (5) are components of the sole (1), and pressure and temperature sensors (3) are components of the lateral elements (2), and
- an energy source (6) connected to the data-processing system (4) and to the transmitter (5), either as a component of the sole (1) or having a securing device for a body part or item of clothing.

2. Protective device according to claim 1, **characterised in that** the sole (1) consists of a support (10) for at least pressure and temperature sensors (3), the data-processing system (4), the transmitter (5) and electrical conductors, and of a cover body (11) on the support (10), pressure and temperature sensors (3), the data-processing system (4), the transmitter (5) and electrical conductors, and **in that** the lateral elements (2) consist of the support (10) for at least pressure and temperature sensors (3) and electrical conductors and of the cover body (11) on the support (10), pressure and temperature sensors (3) and electrical conductors.

3. Protective device according to claim 2, **characterised in that** the support (10) and the cover body (11) consist of a plastics material and/or are textiles and/or leather.

4. Protective device according to claim 2 and 3, **characterised in that** the support (10) and the cover body (11) are stitched or adhesively bonded to one another.

5. Protective device according to claim 4, **characterised in that** the seams or adhesive joints that connect the support (10) and the cover body (11) are designed and arranged such that a seam or adhesion positions a sensor (3) as a pressure and/or temperature sensor (3).

6. Protective device according to claim 1, **characterised in that** the energy source (6) is an accumulator (6) and **in that** the accumulator (6) is connected to an plug-in connector (9) for detachable connection to a charger.

7. Protective device according to claim 6, **characterised in that** the accumulator (6) is connected via a charger to a device that converts mechanical energy or kinetic energy into electrical energy.

8. Protective device according to claim 1, **characterised in that** the data-processing system (4) and the transmitter (5) are interconnected such that, if at least one limit value is not met and/or is exceeded, the transmitter (5) transmits at least one signal in the form of electromagnetic radiation to a receiver (7) for signalling.

9. Protective device according to claim 8, **characterised in that** the data-processing system (4) and the transmitter (5) are interconnected such that signalling takes place when an upper limit pressure value is exceeded at least at one pressure sensor (3) over a time period and there is subsequently no pressure reduction at said pressure sensor (3) below a lower limit value over a time period.

10. Protective device according to claim 8, **characterised in that** the receiver (7) is connected to at least one signal generator (8) for acoustic signals, optical signals, mechanical signals or a combination thereof.

11. Protective device according to claim 1, **characterised in that** a sensor for measuring the ambient temperature is connected to the data-processing system (4), said sensor being located in a housing that is mechanically connected to the sole by means of a resilient connecting element.

12. Method for protecting a diabetic foot from a lack of circulation, wherein the pressure is measured on the foot sole and on the lateral regions of the foot by means of pressure sensors (3) in a sole (1) and lateral elements (2) secured thereto, wherein, at the same time, temperatures that can be associated with the particular blood circulation can be detected by means of temperature sensors (3), and wherein the measured values are compared by means of a data-processing system (4) connected to the pressure and temperature sensors (3), wherein signalling takes place when there is an increase in pressure accompanied by a fall in temperature over a time period at at least one of the pressure and temperature sensors (3).

13. Method according to claim 12, wherein the measured values are compared, by means of the data-processing system (4) connected to the pressure and temperature sensors (3), with stored values, as limit pressure values, in the data-processing system (4), such that signalling takes place when at least one limit pressure value is exceeded over a time period and there is a fall in temperature at at least one of the pressure and temperature sensors (3).

## Revendications

1. Dispositif de protection pour le pied diabétique avec les caractéristiques suivantes
- une semelle (1) qui comporte des parties latérales (2) comportant chacune des capteurs de pression et de température (3) pour les surfaces de la plante du pied afin de mesurer les charges verticales, et pour les parties latérales du pied afin de mesurer les charges horizontales incluant les températures des zones respectives du pied liées à la circulation sanguine,
- un système de traitement de données (4) interconnecté avec les capteurs de pression et de température (3) pour comparer les valeurs mesurées aux valeurs limites sauvegardées, un signal se produisant quand une valeur de pression franchit une valeur limite de pression sur un certain laps de temps au niveau d'au moins un capteur de pression (3) et/ou la température baissant au niveau d'au moins un capteur de température (3), et aucun signal ne se produisant quand la pression baisse sous la valeur de pression au niveau de ce capteur de pression (3) et/ou la température augmentant sous l'effet de la circulation sanguine au niveau de ce capteur de température,
- un émetteur (5) interconnecté avec le système de traitement de données (4), les capteurs de pression et de température (3), le système de traitement de données (4) et l'émetteur (5) étant des composants de la semelle (1) et les capteurs de pression et de température (3) étant des composants des parties latérales (2), et
- une source d'énergie (6) reliée au système de traitement de données (4) et à l'émetteur (5) soit en tant que composant de la semelle (1) soit pour une partie du corps ou un vêtement avec un dispositif de fixation.

2. Dispositif de protection selon la revendication 1, **caractérisé en ce que** la semelle (1) est constituée d'un support (10) pour au moins les capteurs de pression et de température (3), le système de traitement de données (4), l'émetteur (5) et les conducteurs électriques, et d'un corps de couvercle (11) sur le support (10), de capteurs de pression et de température (3), du système de traitement de données (4), de l'émetteur (5) et de conducteurs électriques, et **en ce que** les parties latérales (2) sont constituées du support (10) pour au moins des capteurs de pression et de température (3) et des conducteurs électriques et du corps de couvercle (11) sur le support (10), des capteurs de pression et de température (3) et des conducteurs électriques.

3. Dispositif de protection selon la revendication 2, **caractérisé en ce que** le support (10) et le corps de couvercle (11) sont constitués d'une matière plastique et/ou de textile et/ou de cuir.

4. Dispositif de protection selon les revendications 2 et 3, **caractérisé en ce que** le support (10) et le corps de couvercle (11) sont cousus ou collés ensemble.

5. Dispositif de protection selon la revendication 4, **caractérisé en ce que** les coutures et les surfaces de collage reliant le support (10) et le corps de couvercle (11) sont constituées et disposées de telle sorte qu'une couture ou un collage positionne un capteur (3) comme capteur de température et de pression (3).

6. Dispositif de protection selon la revendication 1, **caractérisé en ce que** la source d'énergie (6) est un accumulateur (6) et que l'accumulateur (6) est relié à un connecteur à fiche (9) pour une connexion amovible à un chargeur.

7. Dispositif de protection selon la revendication 6, **caractérisé en ce que** l'accumulateur (6) est relié via un chargeur à un dispositif de conversion d'énergie mécanique ou d'énergie cinétique en énergie électrique.

8. Dispositif de protection selon la revendication 1, **caractérisé en ce que** le système de traitement de données (4) et l'émetteur (5) sont reliés entre eux de telle sorte que l'émetteur (5) émet au moins un signal sous forme de rayonnement électromagnétique à un récepteur (7) pour le signal en cas de sous-dépassement ou de dépassement d'au moins une valeur limite.

9. Dispositif de protection selon la revendication 8, **caractérisé en ce que** le système de traitement de données (4) et le transmetteur (5) sont interconnectés de telle sorte qu'un signal se produit quand une valeur limite supérieure de pression est dépassée au niveau d'au moins un capteur de pression (3) sur un laps de temps et que par la suite aucune décompression ne se produise en-dessous d'une valeur limite inférieure sur un laps de temps au niveau de ce capteur de pression (3).

10. Dispositif de protection selon la revendication 8, **caractérisé en ce que** le récepteur (7) est relié à au moins un générateur de signaux (8) pour des signaux acoustiques, des signaux optiques, des signaux mécaniques ou une combinaison de ceux-ci.

11. Dispositif de protection selon la revendication 1, **caractérisé en ce qu'**un capteur pour mesurer la température ambiante est relié au système de traitement de données (4), ce capteur étant situé dans un boîtier relié mécaniquement à la semelle par une pièce de liaison élastique.

12. Procédé de protection du pied diabétique contre une mauvaise circulation sanguine, la pression étant mesurée au niveau de la plante du pied et des parties latérales du pied avec des capteurs de pression (3) dans une semelle (1) qui comporte des parties latérales (2), en même temps au moyen de capteurs de température (3) des températures liées à la circulation sanguine respective étant recueillies et les valeurs mesurées étant comparées à un système de traitement de données (4) relié aux capteurs de pression et de température (3), le signal se produisant en cas de pression croissante combinée à une diminution de la température sur un laps de temps au niveau d'au moins un des capteurs de pression et de température (3).

13. Procédé selon la revendication 12, les valeurs mesurées étant comparées avec le système de traitement de données (4) relié aux capteurs de pression et de température (3) avec valeurs sauvegardées en tant que valeurs limite de pression dans le système de traitement de données (4), de telle sorte qu'un signal se produit en cas de dépassement d'au moins une valeur limite de pression sur un laps de temps associé à une température décroissante au niveau d'au moins un élément parmi les capteurs de pression et de température (3).
